# EUROPEAN PATENT SPECIFICATION

(11) **EP 0 537 895 B1**
(45) Date of publication and mention of the grant of the patent: **20.11.1996**
(21) Application number: 92308223.4
(22) Date of filing: 10.09.1992
(51) Int. Cl.: A61M 29/02

(54) **Angioplasty balloon catheter**
Ballonkatheter für Angioplastik
Cathéter à ballon pour angioplastie

(30) Priority: 16.09.1991 US 760723
(43) Date of publication of application: 21.04.1993
(73) Proprietor: Cook Incorporated, Bloomington Indiana 47402 (US)
(72) Inventor: Parker, Fred T., Unionville, Indiana 47468 (US)
(74) Representative: Johnston, Kenneth Graham

(56) References cited:
- EP-A- 0 470 925
- WO-A-89/02763

## Description

This invention relates to angioplasty balloon catheters.

Angioplasty balloon catheters are commonly introduced through a guiding catheter to a treatment site in a vessel of the vascular system, such as a coronary vessel. To move catheters safely into delicate coronary vessels, it has been proposed to use soft distal tips which minimize the risk of causing trauma. In addition, a soft distal tip tracks over a wire guide much easier than a catheter without a soft tip.

The outside diameter of an angioplasty balloon catheter is preferably as small as possible for insertion through tortuous coronary vessels and delivering angioplasty treatment thereto.

WO-89-02763 describes a balloon catheter as is shown in the preamble of claim 1.

According to the present invention there is provided a catheter as defined in claim 1.

The distal end of the angioplasty balloon is attached about, or in the region of, the proximal end of the tubular tip and the proximal end of the balloon is attached about the main portion, proximally from the tip. The uninflated balloon is positioned and stored around a reduced diameter distal portion of the main tubular portion.

The external taper of the soft tip has a maximum outside diameter approximating the outside diameter of the main tubular portion with the uninflated balloon positioned around the reduced diameter distal portion.

The maximum outside diameter of the tubular tip and distal end of the balloon approximate the outside diameter of the main tubular portion with the uninflated balloon positioned therearound.

The balloon further includes a mid-section stored around a reduced diameter distal portion of the main tubular portion and expandable to a fourth outside diameter for dilating a vessel during an angioplasty procedure.

A single lumen tube is smaller in diameter than the dual lumen tube, which allows for storage of the uninflated balloon therearound. The diameter of the stored balloon is advantageously no greater than the dual lumen tube or the soft catheter tip. The second lumen of the dual lumen tube communicates with the interior of the balloon for inflation of the balloon.

### Brief description of the drawings

FIG.1 depicts an embodiment catheter of the invention; and
FIG.2 depicts a cross-sectional view of the proximal portion tube of the catheter of Fig.1.

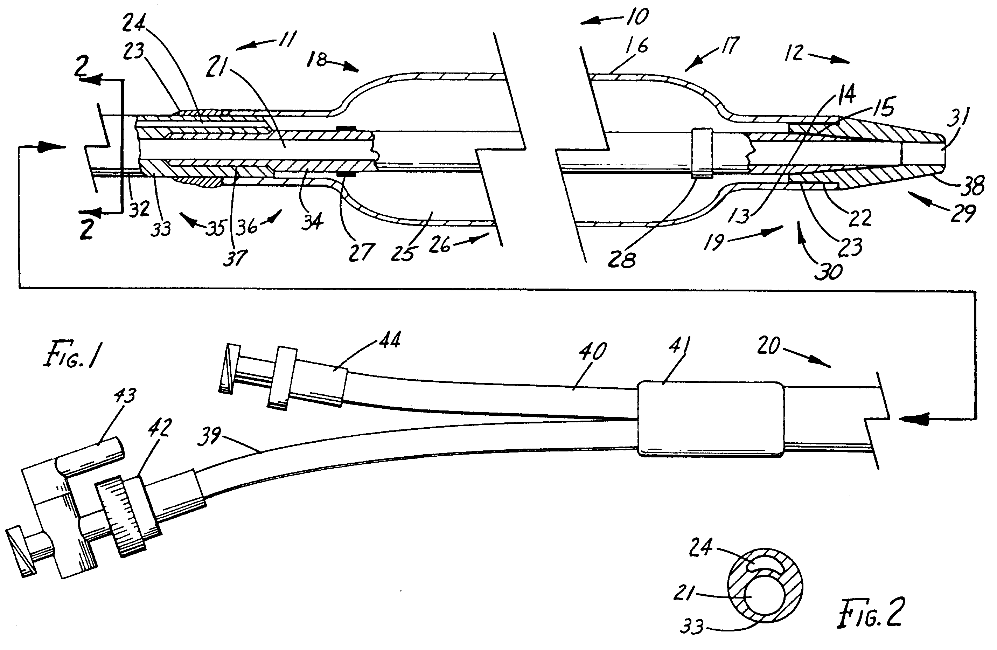

FIG.1 depicts a partially sectioned side view of an illustrative soft tip angioplasty balloon catheter 10 having main tubular portion 11 and soft tubular tip 12 interconnectd by thermal bond 15. Distal end 19 of the main portion includes an external taper 13, and proximal end 30 of the soft tip includes mating internal taper 14. The mating external and internal tapers increase the contact surface area between the main portion and soft tip, thereby strengthening the thermal bond and preventing separation of the main portion and soft tip. The bonded tapers also provide a gradual change in the durometer of the catheter between the main portion and soft tip. Soft tip 12 comprises a soft vinyl material with a durometer softer than the materials of main portion 11. Main portion 11 comprises a harder durometer, vinyl radiopaque material and advances the catheter through the tortuous coronary vessels while the softer durometer tip minimizes vessel wall trauma. The soft tip also increases trackability of the catheter over a wire guide.

The catheter also includes balloon 16 (depicted in an inflated state) having a distal end 17 attached about proximal end 30 of the soft tip in annular recess 22. Distal end 17 of the balloon is positioned in annular recess 22 and attached thereto using, for example, a medical grade adhesive 23 such as Loctite No.454 adhesive. Proximal end 18 of the balloon is attached circumferentially around outer surface 32 of the main portion proximally from the soft tip using, again, medical grade adhesive 23. This normally presents the maximum outside diameter of the main portion with balloon 16 uninflated. The maximum outside diameter of the soft tip with distal end 17 of the balloon attached thereto approximates the maximum outside diameter of the main portion with the uninflated balloon positioned therearound. The approximate diameters provide for ready and atraumatic insertion of the catheter through a guiding catheter and into a coronary vessel.

Main tubular portion 11 of the catheter includes a dual lumen proximal portion tube 33 and a single lumen distal portion tube 34. The dual lumen proximal portion tube comprises a vinyl radiopaque material having a circularly shaped passageway 21 and a second, crescent or quarter-moon shaped passageway 24, as depicted in FIG.2. The vinyl radiopaque material of proximal portion tube 33 has a durometer of 97 on the Shore A scale. The single lumen distal portion tube also comprises a vinyl radiopaque material having a somewhat harder durometer than the proximal portion tube, such as 51 on the Shore D scale. Distal end 35 of proximal portion tube 33 is counterbored and internally tapered, and the outside diameter of proximal end 36 of distal portion tube 34 is reduced and externally tapered as shown. The reduced diameter and externally tapered proximal end of the single lumen tube is inserted into the circular passageway of counterbored and internally tapered distal end 35 and heated to form thermal bond 37.

Main tubular portion 11 is approximately 130cms in length with circularly shaped passageway 21 extending longitudinally therethrough. External taper 13 about distal end 19 extends longitudinally for a predetermined length in a range of 3 to 5mms. Single lumen distal portion tube 34 comprises a 52mms length of 5 French tube with an inside diameter of approximately 0.109cms (0.043") and an outside diameter of approximately 0.168cms (0.066").

Main tubular portion 11 further includes a 100cms length of 5.3 French dual lumen proximal portion tube 33 with circularly shaped passageway 21 and crescent-shaped passageway 24 extending longitudinally therethrough between proximal end 20 and distal end 35. As previously described, circularly shaped passageway 21 extends entirely through main tubular portion 11 for insertion over a wire guide, whereas crescent-shaped passageway 24 extends only through the proximal portion tube to balloon interior 25 for inflating the uninflated balloon with fluid during an angioplasty procedure. When the balloon is inflated, mid-section 26 of the balloon expands for dilating a stenosis in a coronary vessel.

Balloon 16 comprises, for example, polyethylene terephalate material and is approximately 40mms in length with reduced diameter distal and proximal ends 17 and 18, each approximately 4mms in length. The distal and proximal balloon ends are attached respectively to proximal end 30 of the soft tip and distal end 35 of the proximal portion tube for a length of approximately 2mms by commercially available, medical grade adhesive 23. A vinyl dispersion coating (not shown) is placed over the proximal balloon end where it is bonded to the main portion for further affixing the balloon to the main portion and for providing a smooth outer surface at the bond. Balloon markers 27 and 28 are positioned around the main portion under the balloon near the distal and proximal balloon ends, respectively, for providing radiopaque visualization of each end of the mid-section of the balloon. The markers comprise, for example, tungsten rings.

Soft tubular tip 12 comprises a soft vinyl material having a durometer of approximately 60 on the Shore A scale and includes distal end 29, proximal end 30, and passageway 31 extending longitudinally therethrough. Soft tip passageway 31 communicates directly with main tubular portion passageway 21. The soft tubular tip is approximately 6mms in length and has a minimum inner diameter of approximately 0.094cms (0.037") and a maximum outer diameter of approximately 0.2cms (0.08"). The soft tubular tip further includes external taper 38 about distal end 29 for presenting a reduced diameter, blunt, atraumatic surface to vessel walls. External taper 38 extends for a predetermined length in a range of 3 to 5mms and overlaps external taper 13 of the main portion for a predetermined length in a range of 1.5 to 2.5mms. Annular recess 22 positioned about proximal end 30 of the soft tubular tip extends longitudinally for approximately 2mms for positioning and attaching distal end 17 of the balloon thereto.

Proximal end 20 of the main tubular portion is fixedly attached in a well-known manner to single lumen extension tubes 39 and 40 at manifold cover 41 by thermal bonding and an application of a medical grade adhesive. Extension tube 39 comprises, for example, vinyl non-radiopaque tubing approximately 12.5cms in length and has well-known female Luer lock connector 42 fixedly attached about the proximal end thereof using medical grade adhesive. A commercially available one-way stopcock 43 is attached to the female Luer lock connector for controlling the injection and withdrawal of fluid to the interior of the balloon. Extension tube 40 comprises, for example, vinyl nonradiopaque tubing approximately 7cms in length and has well-known female Luer lock connector 44 fixedly attached about the proximal end thereof using a medical grade adhesive.

The dimensions of the above-described catheter are more particularly suited for use in the renal and peripheral vasculature. Another contemplated use of this angioplasty catheter is in the iliac and femoral vessels. Various other sizes of the above-described catheter are contemplated for use in other vessels, such as the coronary and cerebral vessels. It is contemplated that various other materials of comparable durometers may be utilized for the main portion or soft tip. In summary, the soft tip thermally bonded to the main portion by respective internal and external tapers presents a soft, atraumatic surface to delicate coronary vessels.

## Claims

1. A soft tip angioplasty balloon catheter (10) comprising a main tubular portion (34) with a passageway (21) extending longitudinally therethrough, the main portion comprising a first material having a first durometer, a tubular tip (12) comprising a second material having a second durometer softer than the first material, said tip having a distal end (29) and a proximal end (30) with a passageway (31) extending therebetween and in communication with the first mentioned passageway, the main portion and the soft tip having overlapping sections which are bonded together, the soft tip having a section extending distally beyond the distal end of the said tubular portion, an inflatable balloon (16) with the distal end (17) of the balloon attached about or in the region of the proximal end of the soft tip, and with the proximal end (18) of the balloon attached about the said main portion, characterised in that the main portion and the soft tip have cooperating tapered surfaces (13,14) bonded together to form the said overlapping sections, in that the cooperating tapered surfaces extend beyond the distal end of the balloon, and in that the distal end of the tubular tip has an externally tapered section (38).

2. The catheter of claim 1, characterised in that the said externally tapered section (38) partially overlaps the said cooperating tapered surfaces (13,14).

3. The catheter of claim 1, or 2, characterised in that an annular recess (22) is formed at the proximal end of the soft tip for accommodating the distal end of the balloon, with or without adhesive.

4. The catheter of claim 1,2, or 3, characterised in that the balloon comprises polyethylene terephthalate, and/or the main portion includes a vinyl radiopaque tube, and/or the tubular tip comprises soft vinyl material.

5. The catheter of any one preceding claim, characterised in that the main portion is externally tapered (13) about the distal end thereof, and in that the soft tip is internally tapered (14) from the proximal end thereof.

6. A catheter according to any preceding claim, characterised in that the soft tip has a maximum outside diameter adjacent the proximal end thereof, and in that an outer diameter of the tubular portion approximates said maximum diameter.

## Patentansprüche

1. Ballonkatheter (10) mit weicher Spitze für Angioplastie, umfassend einen rohrförmigen Hauptteil (34) mit einem sich in Längsrichtung durch ihn hindurch erstreckenden Durchgang (21), wobei der Hauptteil aus einem ersten Material einer ersten Durometer-Härte besteht, eine rohrförmige Spitze (12), die aus einem zweiten Material einer zweiten Durometer-Härte besteht, das weicher ist als das erste Material, wobei besagte Spitze ein distales Ende (29) und ein proximales Ende (30) mit einem sich dazwischen erstreckenden und mit dem besagten ersten Durchgang in Verbindung stehenden Durchgang (31) aufweist, wobei der Hauptteil und die weiche Spitze sich überlappende, miteinander verbundene Abschnitte aufweisen und die weiche Spitze einen sich distal über das distale Ende des besagten rohrförmigen Teils hinaus erstreckenden Abschnitt aufweist, einen aufblasbaren Ballon (16), dessen distales Ballonende (17) um das proximale Ende der weichen Spitze herum oder in deren Bereich befestigt ist, und dessen proximales Ballonende (18) um besagten Hauptteil befestigt ist, dadurch gekennzeichnet, daß der Hauptteil und die weiche Spitze zusammenwirkende verjüngte Oberflächen (13, 14) aufweisen, die zur Bildung besagter sich überlappender Abschnitte miteinander verbunden sind, daß die zusammenwirkenden verjüngten Oberflächen sich über das distale Ende des Ballons hinaus erstrecken, und daß das distale Ende der rohrförmigen Spitze einen außen verjüngten Abschnitt (38) aufweist.

2. Katheter gemäß Anspruch 1, dadurch gekennzeichnet, daß besagter außen verjüngter Abschnitt (38) besagte zusammenwirkende verjüngte Oberflächen (13, 14) teilweise überlappt.

3. Katheter gemäß Anspruch 1 oder 2, dadurch gekennzeichnet, daß am proximalen Ende der weichen Spitze eine ringförmige Nische (22) zur Aufnahme des distalen Endes des Ballons mit oder ohne einem Klebmittel gebildet ist.

4. Katheter gemäß Anspruch 1, 2 oder 3, dadurch gekennzeichnet, daß der Ballon aus Polyethylenterephthalat besteht und/oder der Hauptteil einen strahlenundurchlässigen Vinylschlauch enthält und/oder die rohrförmige Spitze aus einem weichen Vinylmaterial besteht.

5. Katheter gemäß einem der vorhergehenden Ansprüche, dadurch gekennzeichnet, daß der Hauptteil um sein distales Ende herum außen verjüngt (13) ist und daß die weiche Spitze vom proximalen Ende her innen verjüngt (14) ist.

6. Katheter gemäß einem der vorhergehenden Ansprüche, dadurch gekennzeichnet, daß die weiche Spitze neben dem proximalen Ende einen maximalen Außendurchmesser aufweist und daß ein Außendurchmesser des rohrförmigen Teils nahezu dem besagten maximalen Durchmesser entspricht.

## Revendications

1. Cathéter à ballon pour angioplastie (10) à pointe souple comprenant une portion principale tubulaire (34) avec un passage (21) s'étendant longitudinalement à travers elle, la portion principale comprenant un premier matériau ayant une première valeur de duromètre, une pointe tubulaire (12) comprenant un second matériau, ayant une seconde valeur de duromètre, plus souple que le premier matériau, ladite pointe ayant une extrémité distale (29) et une extrémité proximale (30) avec un passage (31) s'étendant entre elles et communiquant avec le premier passage mentionné, la portion principale et la pointe souple ayant des sections se chevauchant, qui sont collées ensemble, la pointe souple ayant une section s'étendant distalement au-delà de l'extrémité distale de ladite portion tubulaire, un ballon gonflable (16) dont l'extrémité distale (17) est attachée autour ou dans la région de l'extrémité proximale de la pointe souple, et dont l'extrémité proximale (18) est attachée autour de ladite portion principale, caractérisé en ce que la portion principale et la pointe souple ont des surfaces effilées coopérantes (13, 14) collées ensemble pour former lesdites sections se chevauchant, en ce que les surfaces effilées coopérantes s'étendent au-delà de l'extrémité distale du ballon, et en ce que l'extrémité distale de la pointe tubulaire a une section effilée extérieurement (38).

2. Cathéter selon la revendication 1, caractérisé en ce que ladite section effilée extérieurement (38) chevauche partiellement lesdites surfaces effilées coopérantes (13,14).

3. Cathéter selon la revendication 1 ou 2, caractérisé en ce qu'un retrait annulaire (22) est formé à l'extrémité proximale de la pointe souple pour recevoir l'extrémité distale du ballon, avec ou sans adhésif.

4. Cathéter selon la revendication 1, 2 ou 3, caractérisé en ce que le ballon comprend du polyéthylène téréphtalate, et/ou la portion principale comporte un tube vinylique radio-opaque, et/ou la pointe tubulaire comprend un matériau vinylique souple.

5. Cathéter selon l'une quelconque des revendications précédentes, caractérisé en ce que la portion principale est effilée extérieurement (13) autour de son extrémité distale, et en ce que la pointe souple est effilée intérieurement (14) à partir de son extrémité proximale.

6. Cathéter selon l'une quelconque des revendications précédentes, caractérisé en ce que la pointe souple a un diamètre extérieur maximum dans la partie adjacente à son extrémité proximale, et en ce qu'un diamètre extérieur de la portion tubulaire est approximativement le même que ledit diamètre maximum.
